# EUROPEAN PATENT SPECIFICATION

(11) **EP 1 370 322 B1**
(45) Date of publication and mention of the grant of the patent: **09.11.2005**
(21) Application number: 02719156.8
(22) Date of filing: 08.03.2002
(51) Int. Cl.: A61N 1/05

(54) **LEAD WITH ADJUSTABLE ANGULAR AND SPATIAL RELATIONSHIPS BETWEEN ELECTRODES**
LEITUNG MIT ZWISCHEN ELEKTRODEN EINSTELLBAREN WINKEL- UND RAUMPOSITIONEN
LEAD WITH ADJUSTABLE ANGULAR AND SPATIAL RELATIONSHIPS BETWEEN ELECTRODES

(30) Priority: 08.03.2001 US 274364 P
(43) Date of publication of application: 17.12.2003
(73) Proprietor: MEDTRONIC, INC., Minneapolis, Minnesota 55432-5604 (US)
(72) Inventor: CROSS, Thomas, E., Jr., St. Francis, MN 55070 (US)
(74) Representative: Hughes, Andrea Michelle
(86) International application number: PCT/US2002/007024
(87) International publication number: WO 2002/072192

(56) References cited:
- US-A- 3 724 467
- US-A- 4 903 702
- US-A- 5 417 719
- US-A- 6 066 165

## Description

### FIELD OF THE INVENTION

The present invention relates to a medical body implantable lead for electrical stimulation and/or sensing having a series of electrodes at its distal end that are adjustable with respect to each other either axially, spatially or both.

### BACKGROUND OF THE INVENTION

The state of the art of implantable pulse generator and lead systems for stimulating human tissue has advanced to the point that such devices are being designed and used in increasing numbers to treat a wide variety of medical conditions. In addition to implantable pulse generator and lead systems for treating many different types of cardiac conditions (bradycardia, tachycardia, fibrillation, and the like), so called neurological pulse generator and lead systems have been provided for stimulating tissue in a patient's nervous system, in order to treat such diverse conditions as pain, motor impairment, incontinence, and impotence, to name only a few.

In most cases, electrical stimulation pulses are conveyed from an implanted pulse generator to the desired stimulation site by means of an implanted lead having exposed electrodes at its distal end. Typically, implantable spinal cord leads, deep brain leads, brain surface leads or peripheral nerve leads contain multiple electrodes. Two basic styles are available.

One style is the percutaneously inserted lead that is introduced through a Tuohy needle. The implanting physician places the electrode in an appropriate location using fluoroscopic visualization. The procedure is most often done under a local anesthetic. Proper electrode placement is tested using a trial stimulation screening technique to assure that paresthesia is perceived in the affected area. An example of this type of lead is disclosed in U.S. Pat. No. 4,379,462 issued to Borkan.

A typical percutaneous lead has a circular cross section and is fitted with one or more ring electrodes. These ring electrodes usually have an outer surface that is isodiametric with respect to the remainder of the lead. The isodiametric configuration minimizes the difficulty in passing the lead through a vein or through tissue. The smooth surface also minimizes the formation of potentially harmful thrombi when the lead is implanted.

The second basic stimulation lead style is commonly referred to as a "paddle" lead since it typically has a flat planar shape that resembles a paddle. This type of lead is usually surgically implanted through a laminotomy. An example of this type of "paddle" lead is the RESUME® lead manufactured by Medtronic, Inc. of Minneapolis, Minn., the assignee of the present invention. This lead has four axially aligned inline electrodes located on the outer surface of an elongate paddle at the distal end of the lead. In most cases the lead is implanted so that the electrodes are aligned with and lie over the midline of the spinal cord or peripheral nerve. Because leads of this type are surgically implanted, the size of the electrodes may be made larger than those of the percutaneously implanted leads.

With this type lead, various electrode combinations may be selected so that the area of stimulation may be moved along the midline of the spinal cord. An example of a surgically implanted lead is disclosed in U.S. Pat. No. 5,417,719 issued to Hull et al., owned by Medtronic, Inc. of Minneapolis, MN, the assignee of the present invention. Another example of a surgically implanted lead is U.S. Pat. No. 3,724,467 issued to Avery et al.

In the recent past there has been considerable interest among physicians, in having the ability to place the electrodes of a medical lead around the perimeter of peripheral nerves rather than placing them in a single row or column along the surface of the nerve parallel to its centerline. Various paddle shapes have been produced (helical shapes for example) to allow the identification and stimulation of fibers within the nerve itself. However, the electrodes have a fixed angular and spatial relationship with one another depending upon their placement on the paddle. The physician chooses the relationship between the electrodes and the desired fibers by rotating the paddle with respect to the nerve and moving it longitudinally along the surface of the nerve.

Similar problems relating to the fixed relationship between electrodes also exist with respect to placing paddle electrodes adjacent other tissues (other than peripheral nerves and the spinal cord). For example, paddle leads may be desired to perform surface brain tissue stimulation.

It would be desirable to have a lead that allows the physician to determine the spacing and the orientation of the electrodes with respect to each other. This problem is in need of a solution.

In addition to stimulation applications, the use of electrodes for sensing various medical conditions and states has the same problems involving spacing and orientation of electrodes with respect to each other.

Document US-A-4 903 702 discloses an implantable medical lead according to the preamble of claim 1.

The invention is defined in claim 1. Any embodiment which is in contradiction to the subject-matter of claim 1 is not part of the invention.

Preferred embodiments will now be described, by way of example only, with reference to the drawings.

### BRIEF DESCRIPTION OF THE DRAWINGS

FIG. 1 is a perspective view of a neurological lead in accordance with the present invention.
FIG. 2 is an enlarged perspective view of the lead of FIG. 1 with the stretchable web removed for clarity.
FIG. 3 is a block diagram of three electrodes and a connecting wire in accordance with the principles of the present invention.
FIG. 4 is a side view of one embodiment electrode node in accordance with the principles of the present invention.
FIG. 5 is a perspective view of an alternate embodiment of the wire connecting two electrode nodes in accordance with the principles of the present invention.
FIG. 6 is a perspective view of an alternate embodiment of the wire connecting two electrode nodes in accordance with the principles of the present invention.
FIG. 7 is a side view of an alternate embodiment of the invention.
FIG. 8 is a perspective view of an alternate embodiment of the invention.
FIG. 9 is a perspective view of an alternate embodiment of the invention.
FIG. 10 is a side view of an alternate embodiment of the invention.
FIG. 11 is a perspective view of an electrode, sleeve and wire in accordance with the principles of the present invention.

### DESCRIPTION OF THE PREFERRED EMBODIMENTS

The following description of the instant invention will be made in the context of the multi-electrode neurological lead illustrated in the drawings. However, it will be apparent that the concepts and principles of this invention may be applied to other tissue stimulating leads as well as to leads used for sensing.

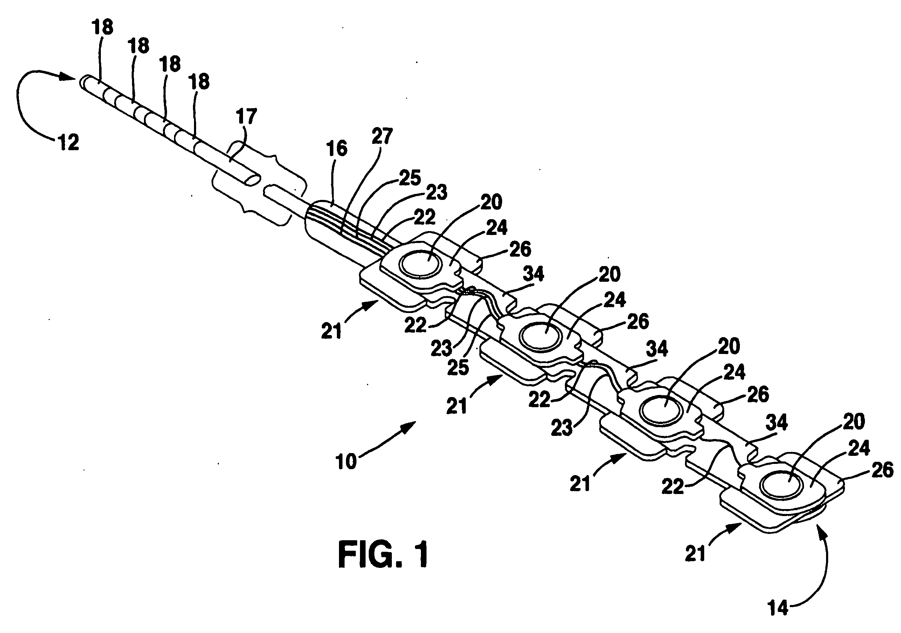

FIG. 1 shows a neural stimulator lead according to one embodiment of the invention generally labeled 10. Lead 10 has a proximal end 12 and a distal end 14. The proximal end 12 of the lead 10 is connected to an electrical signal generator (not shown) either directly or through an extension (not shown). Proximal end 12 terminates in four proximal contacts 18 corresponding to four wires (22, 23, 25, 27), one for each of the four electrodes 20 shown in the embodiment of FIG. 1. The number of contacts and the number of wires may be altered while remaining within the scope of the present invention. The contacts 18 allow the lead to be connected to a connector block (not shown) of a type well known in the art. Each contact 18 is in electrical communication with one of the electrodes 20 at the distal end 14 of lead 10 thereby establishing discrete electrical paths from the electrical signal generator to deliver a stimulating signal to each electrode 20 separately.

It is noted that while there are four conducting wires (22, 23, 25, 27) in lead 10, they may be wrapped together in an outer jacket as is well known in the art. After the most proximal electrode, the three remaining wires (22, 23 and 25) may then be wrapped together in an outer jacket. After the second electrode from the proximal end, the two remaining wires (22, 23) may then be wrapped together in an outer jacket. Only wire 22 remains between the third electrode and the most distal electrode.

Strain relief member 16 connects the lead body 17 to the paddle portion of the lead 10. Strain relief member 16 provides movement flexibility between the lead body 17 and the electrodes 20.

The electrical signal generator may be an implanted device such as an Itrel III® Implantable Pulse Generator (IPG) made and offered for sale by Medtronic, Inc. of Minneapolis, Minnesota. The electrical signal generator may also be a completely external device such as a test stimulator or a partially external and partially internal pulse generators such as the Xtrel® Radio-Frequency Pulse Generator also made and offered for sale by Medtronic, Inc. of Minneapolis, Minnesota.

Lead 10 has at least two electrodes 20. Electrodes 20 are made of an electrically conductive material such as sintered iridium, platinum or a platinum/iridium alloy as is well understood in the art. A typical thickness for an electrode 20 is about 0.005 inch (0.127 mm) although electrodes 20 may be thicker or thinner as is well understood in the art. Electrodes 20 are each connected to the proximal end 12 of lead 10 where they are electrically connected to the electrical signal generator through the proximal contacts 18. In the preferred embodiment, each electrode 20 is connected to a proximal contact 18 through its own wire (22, 23, 25, or 27) as will be described in more detail hereafter.

However, it is within the scope of this invention for each electrode 20 to be connected to an intermediate multiplexing system through a wire 22 before being connected to the proximal end of lead 10. Such a multiplexing system is disclosed in U.S. Pat. No. 6,038,480 entitled "Living Tissue Stimulation and Recording Techniques with Local Control of Active Sites," issued March 14, 2000 to Gregory A. Hrdlicka and Gary W. King.
Electrodes 20 contact the tissue to be electrically stimulated and provide the electrical contact with the tissue.

While the discussion of electrodes 20 above is in the context of providing electrical stimulation, it is important to note that electrodes 20 may also be used for sensing. Sensing applications are within the scope of the present invention.

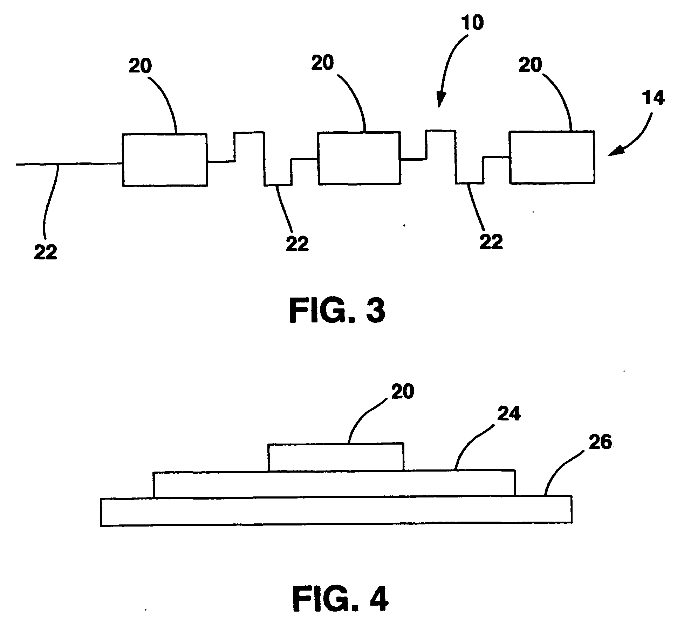

Electrodes 20 are typically located on pads 24. However, pads 24 are not required to be present when tissue contact with the back side of the electrode is acceptable. FIG. 3 shows one embodiment of the invention in which pads 24 are not used. The embodiment of FIG. 3 is also notable because wire 22 connects directly to each electrode. Therefore, in this embodiment the electrodes are not provided with a discrete electrical stimulation but rather are all receiving the same signal.

In one embodiment, a thin mesh 26 is embedded in each pad 24. However, mesh 26 is not required to be present. Mesh 26 may also be connected to the electrodes 20 without the presence of pads 24. In other words, electrodes 20 may be present without both pads 24 and mesh 26 or separately with either pads 24 or mesh 26. The term "electrode node" refers to an electrode alone, or to an electrode plus those components, layers or materials connected to the electrode, but not including the wire or web connecting one electrode to another. For example, an electrode node may be an electrode alone; an electrode and a pad; an electrode, a pad and a mesh; or an electrode, a pad, a mesh and additional layers, components or materials as may be necessary to perform additional functions. One embodiment of an electrode node 21 is shown in FIG. 1. This particular electrode node includes an electrode 20, pad 24 and mesh 26. An electrode node is also shown in FIG. 4.

Pads 24 provide a base for electrodes 20 and allow electrodes 20 to be connected to mesh 26. Pads 24 are preferably made of silicone rubber. However, pads 24 may be made of any suitable insulating material of the types well known in the art. For example, polyurethane or other thermoplastics and polymers such as nylon, polytetrafluoroethylene (PTFE) or the like might used to make pads 24. The particular insulating material used in construction of the pads 24 is not important in the context of this invention so long as the material is a suitable biocompatible polymer that can function as an electrical insulator and with suitable elastic properties. Mesh 26 is preferably made of a medical mesh such as that made of Dacron® and commonly used to anchor medical devices to tissue in the body.

Electrodes 20 may be connected to pads 24 through any well know connection method such as medical adhesives or forming the electrodes in the pads 24 at the time the pads are molded. Pads 24 may be attached to mesh 26 by any well-known connection method such as a medical adhesive or by molding pads 24 around and through the interstices in the mesh 26.

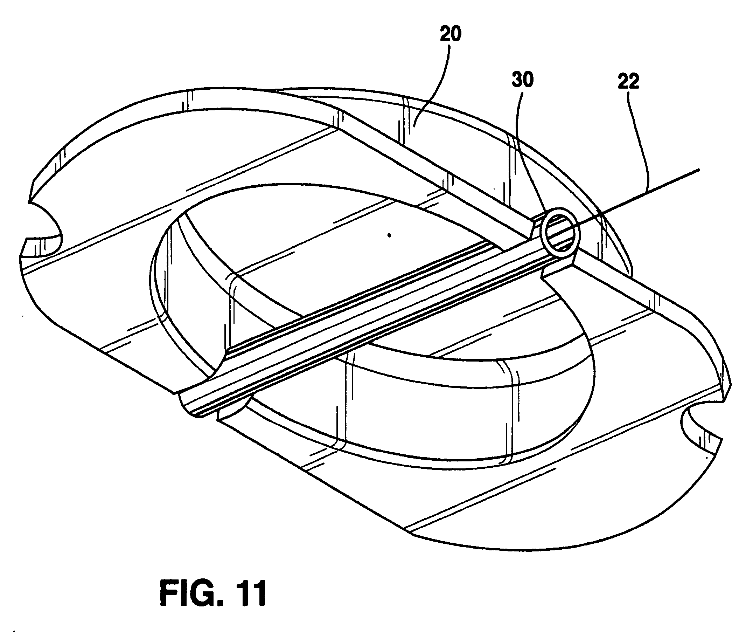

Connection of an electrode 20 to a conducting wire (22, 23, 25, 27) may be accomplished by any way known to connect a wire to another conductor such as an electrode. In one embodiment shown in FIG. 11, a sleeve 30, that may be hollow or partially hollow along its longitudinal direction, is crimped around wire 22. Sleeve 30 is connected to electrode 20 by any means known in the art for connecting two conductors, including laser welding. Other means of connecting a wire (22, 23, 25, 27) to electrode 20 may include soldering, laser welding, conductive adhesives, or other means well known in the art. The connection of electrode 20 to a conducting wire (22, 23, 25, 27) through either sleeve 30, or other means, fully accomplishes the interconnection of electrode 20 with the conducting wire (22, 23, 25, 27) and creates an electrical path from one proximal contact 18 to electrode 20 at the distal end 12 of lead 10.

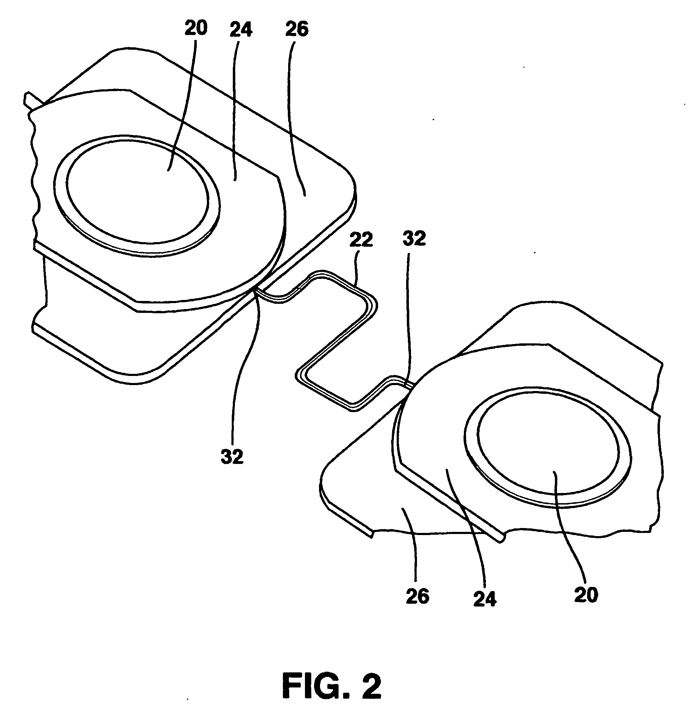

FIG. 2 shows an enlarged view of the last two electrodes on the distal end 14 of the lead 10. In FIG. 2 the silicone rubber web has been removed for clarity. In this embodiment the wire 22 is in the form of a square wave. It is noted that while only one wire 22 is shown in FIG. 2, there may be more than one wire between adjacent electrodes. For example, when it is desired to have discrete electrical signals to each electrode (independent electrodes), then the number of wires between two electrodes would be at least as great as the number of electrodes from that point to the distal end of the lead (one wire for each electrode).

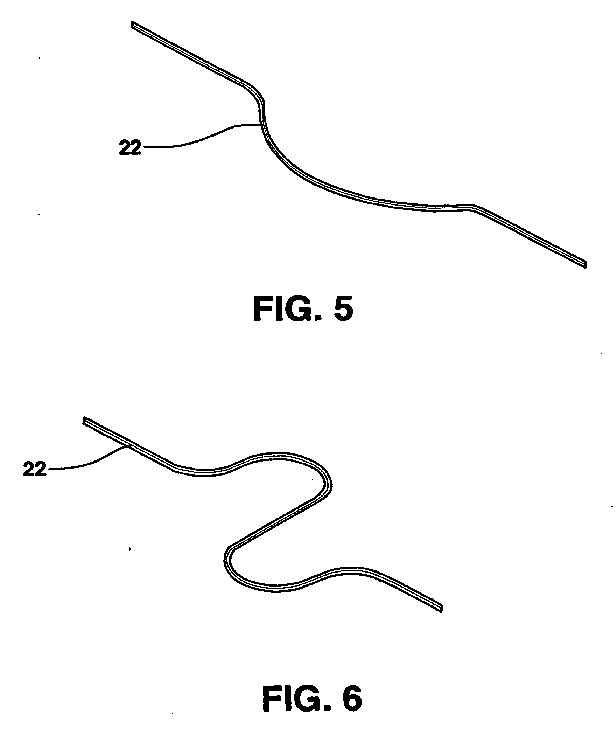

Between electrode nodes 21, wire (22, 23, 25, 27) is a deformable sigma segment. A deformable sigma segment is a segment of wire (22, 23, 25, 27) between endpoints 32 of the wire (22, 23, 25, 27) that has a length greater than the distance between the endpoints 32. A deformable sigma segment may be a square wave as seen in wire 22 of FIG. 2., sine wave (FIG. 6), curve (FIG. 5) or other variation or shape having a length greater than the distance between the endpoints 32 of the wire (22, 23, 25, 27) between pads 24.

Leads 10 having any number of electrodes 20 may be built. For example, configurations of two to any number of electrodes 20 may be desirable. The use of four reference numbers in conjunction with the term "wire" is not meant to be limiting. The FIG. 1 embodiment is just one example that has four electrodes.

Endpoints of a wire such as wire (22, 23, 25, 27) are defined as the first point of contact of the wire to an electrode node. If an electrode node is simply an electrode, then the endpoint is the first point of contact of the wire to the electrode. If an electrode node has other components in addition to an electrode, such as a pad and/or mesh, then the endpoint is the first point of contact of the wire to any of the components of the electrode node. For example, if the wire is embedded in the pad 24, then the endpoint is the first point of contact between the wire and the pad 24.

Depending on the specific medical situation, different types of conducting wire (22, 23, 25, 27) may be used. For example, one type of conducting wire (22, 23, 25, 27) that may be used is a stiff solid wire. Stiff means having sufficient rigidity to be moved into a position and then being able to retain that position. Alternately, wire (22, 23, 25, 27) may be a silver, gold or other highly conductive center-cored wire or bundled stranded wire. It is also within the scope of the invention for wire (22, 23, 25, 27) to be flaccid. Flaccid means having sufficient flexibility that once the wire (22, 23, 25, 27) is moved to a position, little force, such as gravity or contact with a moving surface, is required to move it to another position.

In one embodiment, a thin stretchable web 34 of silicone rubber or similar material connects each pair of pads 24. One or more of electrically conductive wire (22, 23, 25) may be contained within this stretchable web 34. These webs 34, including the wire (22, 23, 25, 27), allow the entire lead 10 to stretch and twist to accommodate a variety of physiological geometries as will be described in more detail hereafter. In a variant of this embodiment, the wire (22, 23, 25, 27) connecting the pads 24 exists without being embedded in or overlaying the stretchable web 34. In a further alternate embodiment, wire (22, 23, 25, 27), whether alone or with web 34, may be provided with an insulated coating, for example, a one mil coating of polytetrafluoroethylene polymer. A typical thickness of such insulation is about 0.0005 inches. The foregoing dimensions are provided for illustrative purposes only and are not intended to limit the scope or spirit of this invention.

It may be desirable to non-surgically place the lead 10 in the body adjacent the tissue to be stimulated. The non-surgical placement of a paddle style lead in the spinal cord area is discussed in U.S. Patent No. 6,249,707 to Kohnen et al.

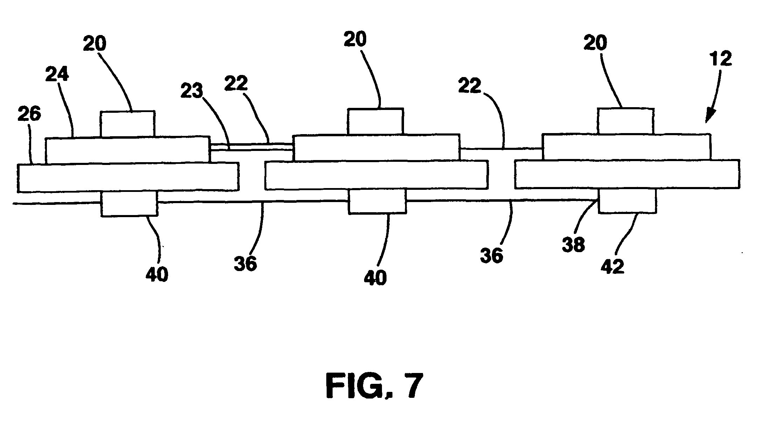

FIG. 7 shows an alternate embodiment of the invention for non-surgical placement of the paddle style lead in the body. In this embodiment, lead 10 includes a stylet 36 having a terminal end 38 that removably extends through cylindrical lumens 40 formed in pad 24 to terminate in a terminal stop 42 at the most distal end 12 of lead 10. Terminal stop 42 is cylindrical with a closed end where the closed end contacts the terminal end 38 of stylet 36. The stylet 36 may be inserted through lumens 40 until the terminal end 38 contacts and engages the terminal stop 42. The stylet 36 stiffens the lead 10 and allows the lead to be moved to a desired location through an oblong cross section of a needle. Once the lead 10 is at the desired location, the stylet 36 may be removed. The stiffening stylet. 36 stiffens the lead 10 during insertion and is intended to be removed after insertion and during use of lead 10.

It is noted that in FIG. 7 a stretchable web is not shown between electrode nodes. However, a stretchable web 34 may be preferred and may be used in conjunction with the embodiment of FIG. 7. The stretchable web 34 shown in FIG. I may be used together with the stylet embodiment of FIG. 7.

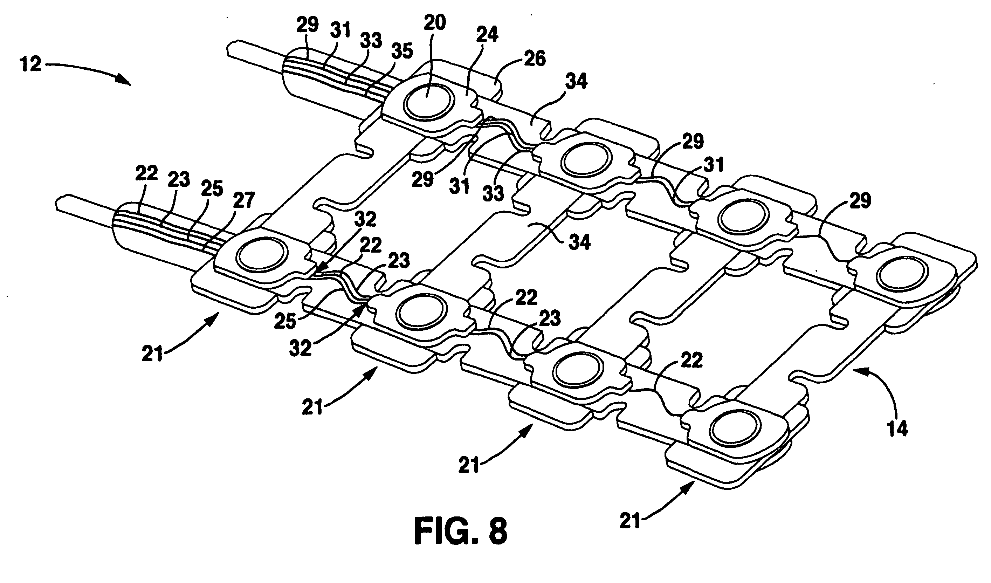

FIG. 8 shows one embodiment of the invention. In this embodiment, lead 10 comprises a series of electrode nodes 21 arranged in a non-linear fashion. Each pad 24 still has an electrode 20. But, pads 24 are interconnected by wires (22, 23, 25, 27, 29, 31, 33,35), webs 34, or both. In the embodiment shown in FIG. 8 wires (22, 23, 25, 27, 29, 31, 33, 35) are each electrically connected to a single electrode 20 and to the source of electrical signal (in the case of stimulation applications). It should be noted however, that many other configurations of wires are within the scope of the present invention. Wires may be included in the design that connect electrode nodes but that do not connect electrodes 20 to the source of electrical signal but only provide the function of connecting electrode nodes together as described above.

In the embodiment of FIG. 8, electrode nodes 21 are arranged in a matrix arrangement so that the lead 10 has a two dimensional configuration. Electrode nodes 21 may be arranged in a series of rows and columns, in circles or concentric circles, along rays from a particular point or points, or otherwise positioned in an infinite variety of configurations. The key to this embodiment is that electrode nodes are connected together by wires with sigma segments and, if desired, stretchable webs 34 in a two dimensional arrangement.

While the embodiment shown in FIG. 8 does not include wires between all electrode nodes, it is certainly within the scope of this invention to have wires between any two electrodes nodes. For example, some of the stretchable webs shown in FIG. 8 do not include an embedded wire. Such stretchable webs may include one or more wires embedded therein or partially embedded therein.

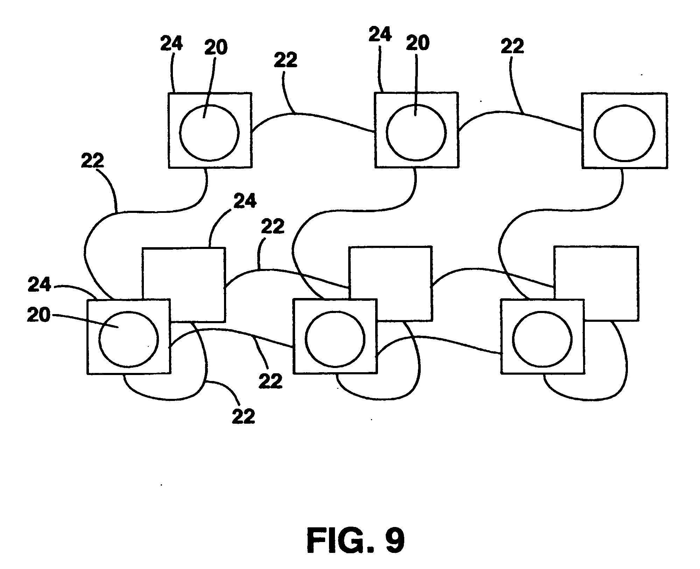

In an alternate embodiment shown in FIG. 9, pads 24 may be arranged in a three dimensional configuration. In this embodiment, pads 24 may also be arranged in a series of rows and columns along three axes, in spheres or concentric spheres, along rays from a particular point or points, or otherwise positioned in an infinite variety of configurations including combinations of two and three dimensional arrays. The key to this embodiment is that electrode nodes are connected together by wires 22 and, if desired, webs 34 in a three dimensional arrangement. Note that in FIG. 9, only a single wire 22 is represented. It should be recognized that multiple wires are likely to be used in this invention to provide separate electrical connections to each electrode 20 as was described above.

In use, in whatever embodiment is used, the physician manipulates the pads 24 to change their relative orientation. In the embodiments where the wires (22, 23, 25, 27) that interconnect the pads 24 are rigid, once the orientation of the pads 24 has been changed, the lead 10 will retain the new orientation. Because wires 22 between pads 24 originally have a length greater than the endpoints 26 of wires 22, the separation of pads 24 from one another may be accomplished by straightening or compressing the wires (22, 23, 25, 27) between respective pads 24. This allows the physician to change not only the relative orientation of the pads 24 but also the spacing between the pads 24 to allow the electrodes 20 to be placed directly on particular sites of interest. Thereafter, if lead 10 is an embodiment where mesh 26 is present, mesh 26 around pads 24 may be sutured, glued or otherwise connected to the tissue near and around the electrodes 20.

In the embodiments where the wires (22, 23, 25, 27) or webs 34 or both that interconnect the pads 24 are flaccid, once the orientation of the pads 24 has been changed, the lead 10 will not retain the new orientation. However, the pads can be affixed to the tissue of interest at particular sites of interest by placing the electrodes 20 in contact with the tissue and suturing, gluing or otherwise affixing the mesh 26 to the tissue. Again, because wires (22, 23, 25, 27) between pads 24 originally have a length greater than the. endpoints 26 of wires (22, 23, 25, 27), the separation of pads 24 from one another or the relative orientation between the pads 24 may be accomplished by straightening, compressing or changing the angles of the wires (22, 23, 25, 27) or webs 34 between respective pads 24. This allows the physician to change not only the relative angular orientation of the pads 24 but also the spacing between the pads 24 to allow the electrodes 20 to be placed directly on particular sites of interest. Thereafter, if lead 10 is an embodiment where mesh 26 is present, mesh 26 around pads 24 may be sutured, glued or otherwise connected to the tissue near and around the electrodes 20.

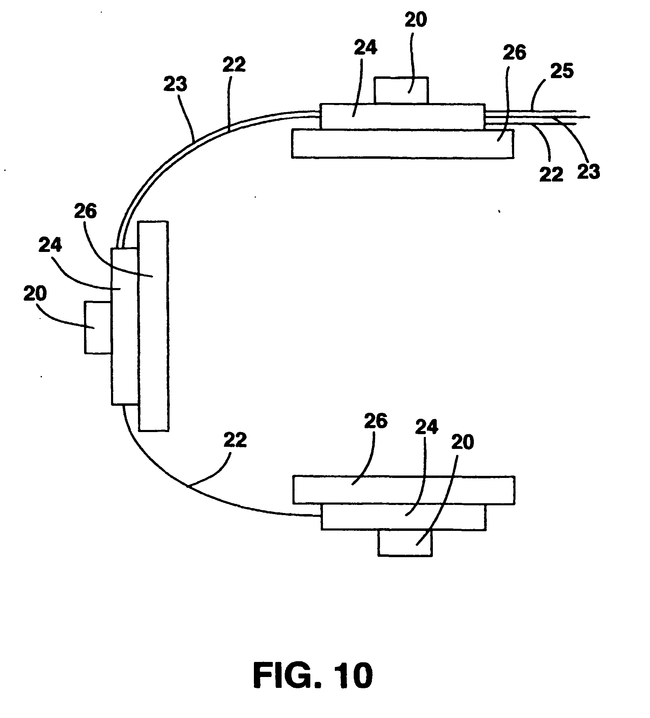

FIG. 10 is another embodiment of the present invention in which three electrode nodes are arranged in an exemplary spatial relationship.

## Claims

1. An implantable medical lead comprising:
at least first and second electrode nodes (21), the first electrode node comprising a first electrode (20) and the second electrode node comprising a second electrode (20); and
at least a first wire (22) connecting the first electrode node to the second electrode node, wherein the first wire is electrically connected to the second electrode, **characterized in that** the first wire defining a deformable sigma segment whereby the deformable sigma segment of the first wire allows the position of the first and second electrode nodes to change relative to each other.

2. An implantable medical lead as in claim 1, further comprising a stretchable web (34) connecting the first electrode node to the second electrode node.

3. An implantable medical lead as in claim 2, wherein the first wire is at least partially embedded in the stretchable web (34).

4. An implantable medical lead as in claim 2 or 3, wherein the stretchable web (34) comprises an elastomer.

5. An implantable medical lead as in claim 4, wherein the elastomer comprises silicone rubber.

6. An implantable medical lead as in claim 2, 3, 4 or 5, wherein the first and second electrode nodes each comprise a pad (24), and wherein each pad is integral with the stretchable web.

7. An implantable medical lead as in claim 1, wherein the first electrode node further comprises a first pad (24) adjacent the first electrode and the second electrode node further comprises a second pad (24) adjacent the second electrode.

8. An implantable medical lead as in claim 6 or 7, wherein the connection between first and second electrode nodes comprises contact of the first wire with the first pad and electrical connection of the first wire to the second electrode.

9. An implantable medical lead as in claim 8 further comprising a second wire, wherein the second wire is electrically connected to the first electrode.

10. An implantable medical lead as in claim 6 or 7, wherein the first and second electrode nodes each further comprise a mesh (26) adjacent one or more of the respective electrode and pad.

11. An implantable medical lead as in any preceding claim, wherein the deformable sigma segment comprises a sine wave form.

12. An implantable medical lead as in any of claims 1 to 10, wherein the deformable sigma segment comprises a half sine wave form.

13. An implantable medical lead as in any of claims 1 to 10, wherein the deformable sigma segment comprises a square wave form.

14. An implantable medical lead as in any preceding claim, wherein the first wire is stiff.

15. An implantable medical lead as in any of claims 1 to 13, wherein the first wire is flaccid.

16. An implantable medical lead as in claim 1 or 2, wherein the first electrode node further comprises a cylindrical lumen, and the second electrode node further comprises a terminal stop (42), and wherein the medical lead further comprises a stylet (36) having a terminal end (38), the stylet capable of being removably extended through the cylindrical lumen, and the terminal end of the stylet capable of contacting the terminal stop.

## Patentansprüche

1. Implantierbare medizinische Leitung mit:
mindestens einem ersten und einem zweiten Elektrodenknoten (21), wobei der erste Elektrodenknoten eine erste Elektrode (20) und der zweite Elektrodenknoten eine zweite Elektrode (20) aufweist, und
mindestens einem ersten Draht (22), der den ersten Elektrodenknoten mit dem zweiten Elektrodenknoten verbindet, wobei der erste Draht elektrisch mit der zweiten Elektrode verbunden ist,
**dadurch gekennzeichnet, dass**
der erste Draht ein verformbares Sigmasegment definiert, wodurch das verformbare Sigmasegment des ersten Drahts ein Ändern der Position des ersten und des zweiten Elektrodenknotens relativ zueinander ermöglicht.

2. Implantierbare medizinische Leitung nach Anspruch 1, die des weiteren ein dehnbares Gewebe (34) aufweist, das den ersten Elektrodenknoten mit dem zweiten Elektrodenknoten verbindet.

3. Implantierbare medizinische Leitung nach Anspruch 2, wobei der erste Draht zumindest teilweise in das dehnbare Gewebe (34) eingebettet ist.

4. Implantierbare medizinische Leitung nach Anspruch 2 oder 3, wobei das dehnbare Gewebe (34) ein Elastomer aufweist.

5. Implantierbare medizinische Leitung nach Anspruch 4, wobei das Elastomer Silikongummi umfasst.

6. Implantierbare medizinische Leitung nach Anspruch 2, 3, 4 oder 5, wobei der erste und der zweite Elektrodenknoten jeweils eine Auflage (24) aufweisen und wobei jede der Auflagen integral mit dem dehnbaren Gewebe ausgebildet ist.

7. Implantierbare medizinische Leitung nach Anspruch 1, wobei der erste Elektrodenknoten des weiteren eine erste Auflage (24) neben der ersten Elektrode aufweist und der zweite Elektrodenknoten des weiteren eine zweite Auflage (24) neben der zweiten Elektrode aufweist.

8. Implantierbare medizinische Leitung nach Anspruch 6 oder 7, wobei die Verbindung zwischen dem ersten und dem zweiten Elektrodenknoten einen Kontakt des ersten Drahts mit der ersten Auflage und eine elektrische Verbindung des ersten Drahts mit der zweiten Elektrode umfasst.

9. Implantierbare medizinische Leitung nach Anspruch 8, die des weiteren einen zweiten Draht aufweist, der elektrisch mit der ersten Elektrode verbunden ist.

10. Implantierbare medizinische Leitung nach Anspruch 6 oder 7, wobei der erste und der zweite Elektrodenknoten jeweils des weiteren ein Netz (26) neben der jeweiligen Elektrode und/oder der jeweiligen Auflage aufweisen.

11. Implantierbare medizinische Leitung nach einem der voranstehenden Ansprüche, wobei das verformbare Sigmasegment eine Sinuswellenform aufweist.

12. Implantierbare medizinische Leitung nach einem der Ansprüche 1 bis 10, wobei das verformbare Sigmasegment eine halbe Sinuswellenform aufweist.

13. Implantierbare medizinische Leitung nach einem der Ansprüche 1 bis 10, wobei das verformbare Sigmasegment eine Rechteckwellenform aufweist.

14. Implantierbare medizinische Leitung nach einem der voranstehenden Ansprüche, wobei der erste Draht steif ist.

15. Implantierbare medizinische Leitung nach einem der Ansprüche 1 bis 13, wobei der erste Draht schlaff ist.

16. Implantierbare medizinische Leitung nach Anspruch 1 oder 2, wobei der erste Elektrodenknoten des weiteren ein zylindrisches Lumen aufweist und der zweite Elektrodenknoten des weiteren einen Anschlussanschlag (42) aufweist und wobei die medizinische Leitung des weiteren einen Führungsstab (36) mit einem Anschlussende (38) aufweist, wobei der Führungsstab entfernbar durch das zylindrische Lumen vorgeschoben werden kann und das Anschlussende des Führungsstabs den Anschlussanschlag berühren kann.

## Revendications

1. Branchement médical implantable comportant :
au moins des premier et second noeuds d'électrode (21), le premier noeud d'électrode comportant une première électrode (20) et le second noeud d'électrode comportant une seconde électrode (20), et
au moins un premier fil (22) reliant le premier noeud d'électrode au second noeud d'électrode, dans lequel le premier fil est électriquement relié à la seconde électrode, **caractérisé en ce que** le premier fil définit un segment sigma déformable par lequel le segment sigma déformable du premier fil permet aux positions des premier et second noeuds d'électrode de changer l'une par rapport à l'autre.

2. Branchement médical implantable selon la revendication 1, comportant de plus une bande extensible (34) reliant le premier noeud d'électrode au second noeud d'électrode.

3. Branchement médical implantable selon la revendication 2, dans lequel le premier fil est au moins en partie intégré dans la bande extensible (34).

4. Branchement médical implantable selon la revendication 2 ou 3, dans lequel la bande extensible (34) comprend un élastomère.

5. Branchement médical implantable selon la revendication 4, dans lequel l'élastomère comporte du caoutchouc de silicone.

6. Branchement médical implantable selon la revendication 2, 3, 4 ou 5, dans lequel les premier et second noeuds d'électrode comportent chacun une plage de connexion (24), et dans lequel chaque plage de connexion est d'un seul tenant avec la bande extensible.

7. Branchement médical implantable selon la revendication 1, dans lequel le premier noeud d'électrode comporte de plus une première plage de connexion (24) adjacente à la première électrode et le second noeud d'électrode comporte de plus une seconde plage de connexion (24) adjacente à la seconde électrode.

8. Branchement médical implantable selon la revendication 6 ou 7, dans lequel la connexion entre les premier et second noeuds d'électrode comporte un contact du premier fil avec la première plage de connexion et une connexion électrique du premier fil à la seconde électrode.

9. Branchement médical implantable selon la revendication 8, comportant de plus un second fil, dans lequel le second fil est électriquement connecté à la première électrode.

10. Branchement médical implantable selon la revendication 6 ou 7, dans lequel les premier et second noeuds d'électrode comportent de plus chacun une maille (26) adjacente à une ou plusieurs des électrodes et plages de connexion respectives.

11. Branchement médical implantable selon l'une quelconque des revendications précédentes, dans lequel le segment sigma déformable constitue une forme d'onde sinusoïdale.

12. Branchement médical implantable selon l'une quelconque des revendications 1 à 10, dans lequel le segment sigma déformable constitue une forme d'onde demi-sinusoïdale.

13. Branchement médical implantable selon l'une quelconque des revendications 1 à 10, dans lequel le segment sigma déformable constitue une forme d'onde carrée.

14. Branchement médical implantable selon l'une quelconque des revendications précédentes, dans lequel le premier fil est rigide.

15. Branchement médical implantable selon l'une quelconque des revendications 1 à 13, dans lequel le premier fil est flasque.

16. Branchement médical implantable selon la revendication 1 ou 2, dans lequel le premier noeud d'électrode comporte de plus une lumière cylindrique, et le second noeud d'électrode comporte de plus une butée terminale (42), et dans lequel le branchement médical comporte de plus un stylet (36) ayant une extrémité terminale (38), le stylet pouvant être étendu d'une manière mobile à travers la lumière cylindrique, et l'extrémité terminale du stylet pouvant être en contact avec la butée terminale.
